# EUROPEAN PATENT SPECIFICATION

(11) **EP 1 403 263 B1**
(45) Date of publication and mention of the grant of the patent: **22.06.2005**
(21) Application number: 02021686.7
(22) Date of filing: 27.09.2002
(51) Int. Cl.: C07D 319/20

(54) **Process for the preparation of N-(2,3-dihydrobenzo 1,4 dioxin-2-carbonyl) piperazine**
Verfahren zur Herstellung von N-2,3-Dihydrobenzo[1,4]dioxin-2-carbosäure-piperazinamid
Procédé pour la préparation de la N-(2,3-dihydrobenzo[1,4]dioxin-2-carbonyl)piperazine

(43) Date of publication of application: 31.03.2004
(73) Proprietor: Council of Scientific and Industrial Research, New Delhi 110 001 (IN)
(72) Inventor: Pardhasaradhi, Malladi, Hyderabad-500 007 Andhra Pradesh (IN); Kumaraswamy, Gullapalli, Hyderabad-500 007 Andhra Pradesh (IN); Kanti Das, Arun, Hyderabad-500 007 Andhra Pradesh (IN); Jena, Nivedita, Hyderabad-500 007 Andhra Pradesh (IN); Kamalakshyamma Snehalatha Nair, Chembumkulam, Hyderabad-500 007 Andhra Pradesh (IN); Naga Venkata Sastry, Mudiganti, Hyderabad-500 007 Andhra Pradesh (IN)
(74) Representative: Henkel, Feiler & Hänzel

(56) References cited:
- FR-A- 2 407 929
- US-A- 4 188 390
- US-A- 5 919 931
- FANG, Q. KEVIN ET AL: "Practical chemical and enzymatic technologies for (S)-1,4-benzodioxan-2- carboxypiperizine intermediate in the synthesis of (S)-doxazosin mesylate" TETRAHEDRON: ASYMMETRY (2001), 12(15), 2169-2174 , XP004308981
- CHOU, WEN-CHIH ET AL: "HMDS-promoted in situ amidation reactions of carboxylic acids and amines" TETRAHEDRON LETTERS (1999), 40(17), 3419-3422 , XP004162298
- CHOU, WEN-CHIH ET AL: "One-Pot Neat Reactions of Carboxylic Esters and Alkylenediamines for Efficient Preparation of N-Acylalkylenediamines" JOURNAL OF ORGANIC CHEMISTRY (1998), 63(26), 10015-10017 , XP002225054
- CAMPBELL, SIMON F. ET AL: "2,4-Diamino-6,7-dimethoxyquinazolines. 1. 2-[4-(1,4-Benzodioxan-2- ylcarbonyl)piperazin-1-yl] derivatives as.alpha.1-adrenoceptor antagonists and antihypertensive agents" JOURNAL OF MEDICINAL CHEMISTRY (1987), 30(1), 49-57 , XP002225055

## Description

### FIELD OF THE INVENTION

The present invention relates to a process for the preparation of N-(2,3- Dihydrobenzo[1,4]dioxin-2- carbonyl) piperazine.

### BACKGROUND OF THE INVENTION

N-(2,3-Dihydrobenzo[1,4]dioxin-2-carbonyl) piperazine is an important intermediate for the preparation of an antihypertensive drug viz., doxazocin. Doxazocin is prepared by reaction of N-(2,3- Dihydrobenzo[1,4]dioxin-2-carbonyl)piperazine with 4-amino-2- chloro-6,7-dimethoxy quinazoline[J. Med. Chem., 1987, 30(1), 49]. However, it was observed that the product obtained by this condensation contained bis-amide as impurity. It makes the product unsuitable for pharmaceutical use as doxazocin base prepared by basification, on analysis showed purity of 91-94% only. Further purification of the base was very difficult due to its polymorphic nature. Large number of references are available mostly of patents (US patents 6,140,334; 6,133,269; 6,130,218) in which doxazocin base has been subjected to tedious purification procedures consisting of four to five steps in order to obtain pure doxazocin of pharmaceutical grade.

Pharmaceutical industry requires pure doxazocin (99.99%) for processing into solid dosage form. In our attempts to prepare pure doxazocin , we found that the purity and crystalline nature of doxazocin were dependent on the purity of N-(2,3-Dihydrobenzo[1,4]dioxin-2-carbonyl) piperazine that was used for the condensation. While it was easy to prepare 4-amino-2-chloro-6,7-dimethoxy quinazoline in high yield and purity, the preparation of N-(2,3-Dihydrobenzo[1,4]dioxin-2-carbonyl)piperazine posed several problems due to its contamination with the bis-amide impurity. It is glassy and difficult to work with. It is essential to evolve a method for the preparation of pure amide not contaminated with bis-amide.

N-(2,3-Dihydrobenzo[1,4]dioxin-2-carbonyl)piperazine is a condensation product of ethyl 2,3- Dihydrobenzo[1,4]dioxin-2-carboxylate and piperazine[eq-1]. N-Acyl alkylenediamines were earlier prepared by the monoamidation of alkylenediamines with acid chlorides. The reaction requires the masking of diamines as monoacetates or hydrogen halides [J. Med. Chem.,1997, 20, 146]. The procedure is tedious and requires i) saponification of ester ii) treatment of the resulting acid with thionyl chloride to give the acid chloride and iii) amidation with piperazine hydrobromide in methanol. There is also one report of the synthesis of N-Acyl alkylenediamines(94% yield) by direct monoamidation of esters[J. Org. Chem., 1998, 63, 10015]. In this procedure, ethyl 2,3- dihydrobenzo[1,4]dioxin-2-carboxylate and piperazine were heated under reflux for 3h. The reaction mixture was partitioned between CHCl₃ and saturated aq. NaHCO₃. The organic layer was washed, dried and concentrated. However, N-(2,3- Dihydrobenzo[1,4]dioxin-2-carbonyl)piperazine prepared by the above method when coupled with 4- amino-2-chloro-6,7-dimethoxy quinazoline gave a product, which on subsequent basification, gave doxazocin base with 92-95% purity. In view of the above, it is necessary to develop an improved process for the preparation of high purity N- (2,3-Dihydrobenzo[1,4]dioxin-2-carbonyl)piperazine without contamination with bis- amide.

### OBJECTS OF THE INVENTION

The main objective of the present invention is to provide a process for the preparation of high purity N- (2,3-Dihydrobenzo[1,4]dioxin-2-carbonyl)piperazine of Formula 1.

Another object of the present invention is to provide N-(2,3-Dihydrobenzo[1,4]dioxin- 2-carbonyl) piperazine free from bis-amide and other impurities by simple acid base work-up procedure.

### SUMMARY OF THE INVENTION

Accordingly, the present invention provides an improved process for the preparation of N-(2,3-Dihydrobenzo[1,4]dioxin-2-carbonyl)piperazine which comprises heating a reaction mixture of 2,3- dihydrobenzo[1,4]dioxin-2-carboxylate and piperazine with a molar ratio of carboxylate to piperazine in the range of 1:2 to 2:1, under nitrogen atmosphere, at a temperature in the range of 70-90 °C for a period of 3-15 hrs, cooling the above said reaction mixture to a temperature of 25-30 °C and dissolving it in chloroform followed by washing with saturated sodium bi-carbonate solution and water respectively, acidifying the organic layer by about 10% HCl to a pH ranging between 1 to 4, separating the organic layer and washing the aqueous layer with chloroform, basifying the aqueous layer with sodium bi-carbonate to a pH ranging between 7 to 9 followed by extracting with chloroform and evaporating the chloroform to obtain the final desired product in amorphous solid form.

In an embodiment of the present invention the reaction mixture is heated for a period preferably in the range of 5 - 12 hrs.

In yet another embodiment the temperature used in heating reaction mixture is preferably in the range of 75 - 80 °C.

In still another embodiment the purity of the compound N-(2,3- Dihydrobenzo[1,4]dioxin-2-carbonyl) piperazine obtain is in the range of 99.3-99.9%.

### DETAILED DESCRIPTION OF THE INVENTION

In the improved process for the preparation of N-(2,3-Dihydrobenzo[1,4]dioxin-2- carbonyl) piperazine, ethyl 2,3-dihydrobenzo[1,4]dioxin-2-carboxylate and piperazine were reacted under a blanket of nitrogen at a temperature preferably in the range of 70- 90 °C for a period preferably in the range of 5-12 h. The reaction mixture on cooling was taken into chloroform. The chloroform layer is acidified to a pH preferably in the range of 4-2. The aqueous layer was basified using solid NaHCO₃ to a pH preferably in the range of 7-8. It is again extracted into chloroform, dried and evaporated to give pure N-(2,3- Dihydrobenzo[1,4]dioxin-2-carbonyl) piperazine as white solid-without any contamination. Pharmaceutical grade doxazocin is prepared with this amide in further process.

The following examples are given by way of illustration of the present invention and therefore should not be construed to limit the scope of the present invention.

### Example-1

Piperazine (101.3g, 1.175 moles) and ethyl 2-carboethoxy-1,4-benzodioxalane (200g, 0.96 moles) were taken into a round bottom flask provided with a stirrer, thermowell, and distillation setup. The reaction mixture was heated for 4 h to reach an internal temperature of about 75 °C while stirring. It was cooled to room temperature, dissolved in chloroform (200ml) and washed with saturated sodium bicarbonate solution (1x150ml) followed by water wash (3x150ml). The organic layer was acidified to pH 2 with 10% HCl (600ml). The organic layer was separated and aqueous layer washed with chloroform (3x100m1). The aqueous portion was basified with solid sodium bicarbonate to pH 8 and was extracted with chloroform (5x150ml). The chloroform layer was evaporated to yield amorphous solid powder (145g, 61% yield), purity 99.9%.

### Example-2

Piperazine (10.13g, 0.1175 moles) and ethyl 2-carboethoxy-1,4-benzodioxalane (20g, 0.096 moles) were taken into a round bottom flask provided with a stirrer, thermowell and distillation setup. The reaction mixture was heated for 10 h to reach an internal temperature of about 75 °C while stirring. It was cooled to room temperature, dissolved in chloroform and washed with saturated sodium bicarbonate solution followed by water. The organic layer was acidified to pH 2 with 10% HCl. The organic layer was separated and aqueous layer washed with chloroform. This aqueous portion was basified with solid sodium bicarbonate to pH 8 and was extracted with chloroform. The chloroform layer was evaporated to yield amorphous solid powder (21.67g, 89.5% yield) purity 99.9%.

### Example-3

Piperazine (8.2g, 0.096 moles) and ethyl 2-carboethoxy-1,4-benzodioxalane (20g, 0.096 moles) were taken into a round bottom flask provided with a stirrer, thermowell and distillation setup. The reaction mixture is heated for 10h to reach an internal temperature of about 75 °C while stirring. It was cooled to room temperature, dissolved in chloroform and washed with saturated sodium bicarbonate solution followed by water. The organic layer was acidified to pH 2 with 10% HCl. The organic layer was separated and aqueous layer washed with chloroform. This aqueous portion was basified with solid sodium bicarbonate to pH 8 and was extracted with chloroform. The chloroform layer was evaporated to yield amorphous solid powder (7.14g, 30% yield), purity 99.3%.

### Example-4

piperazine (12.4g, 0.144 moles) and ethyl 2-carboethoxy-1,4-benzodioxalane (20g, 0.096 moles) were taken into a round bottom flask provided with a stirrer, thermowell and distillation setup. The reaction mixture was heated for 10 h to reach an internal temperature of about 75 °C while stirring. It was cooled to room temperature, dissolved in chloroform and washed with saturated sodium bicarbonate solution followed by water. The organic layer was acidified to pH 2 with 10% HCl. The organic layer was separated and aqueous layer washed with chloroform. This aqueous portion was basified with sodium bicarbonate to pH 8 and was extracted with chloroform. The chloroform layer was evaporated to yield amorphous solid powder (10.7g, 45% yield), purity 99.6%.

### Example-5

Into a round bottom flask provided with a stirrer, thermowell and distillation set up were taken piperazine (5.5g, 0.064 moles) and ethyl 2-carboethoxy-1,4-benzodioxalane (20g, 0.096 moles). The reaction mixture is heated for 10 h to reach an internal temperature of about 75 °C while stirring. It was cooled to room temperature, dissolved in chloroform and washed with saturated sodium bicarbonate solution followed by water. The organic layer was acidified to pH 2 with 10% HCl. The organic layer was separated and aqueous layer washed with chloroform. This aqueous portion was basified with solid sodium bicarbonate to pH 8 and was extracted with chloroform. The chloroform layer was evaporated to yield amorphous solid powder (7.5g,31.5% yield), purity 99.45%.

### The main advantages of the present invention are:

It is a very convenient process in which the bis-amide easily seperated by acid treatment of the reaction mixture of N-(2,3-Dihydrobenzo[1,4]dioxin-2-carbonyl)piperazine.

N-(2,3-Dihydrobenzo[1,4]dioxin-2-carbonyl)piperazine prepared by this method when coupled with 4-amino-2-chloro-6,7-dimethoxy quinazoline, gives the HCl salt which on basification affords 99.99% pure doxazocin base.

The doxazocin base thus obtained can be used without any further purification to get doxzocin mesylate with purity (99.99%) as required by the pharmaceutical industry.

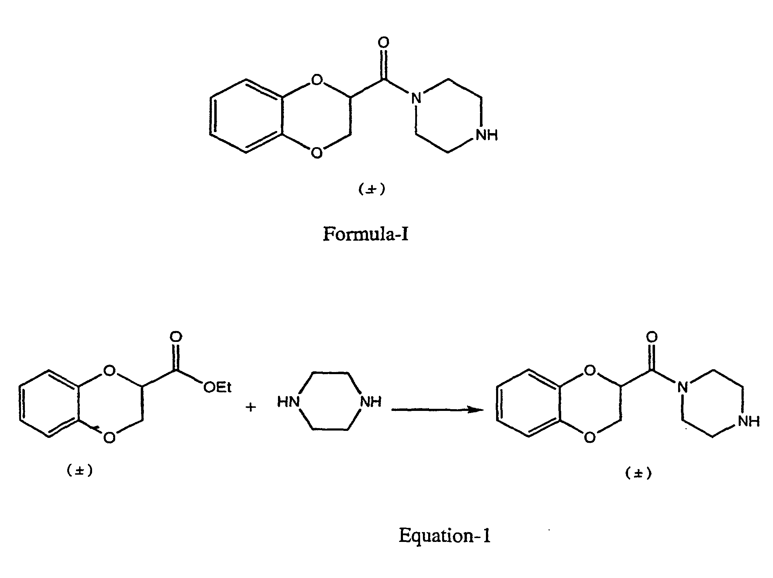

## Claims

1. A process for the preparation of N-(2,3-Dihydrobenzo[1,4]dioxin-2- carbonyl)piperazine which comprises heating a reaction mixture of 2,3- dihydrobenzo[1,4]dioxin-2-carboxylate and piperazine with a molar ratio of carboxylate to piperazine in the range of 1:2 to 2:1, under nitrogen atmosphere, at a temperature in the range of 70-90 °C for a period of 3-15 hrs, cooling the above said reaction mixture to a temperature of 25-30 °C and dissolving it in chloroform followed by washing with saturated sodium bi-carbonate solution and water respectively, acidifying the organic layer by about 10% HCl to a pH ranging between 1 to 4, separating the organic layer and washing the aqueous layer with chloroform, basifying the aqueous layer with sodium bi-carbonate to a pH ranging between 7 to 9 followed by extracting with chloroform and evaporating the chloroform to obtain the final desired product in amorphous solid form.

2. A process as claimed in claim 1, wherein the reaction mixture is heated for a period preferably in the range of 5 - 12 hrs.

3. A process as claimed in anyone of claims 1 and 2, wherein the reaction temperature used is preferably in the range of 75 - 80 °C.

4. A process as claimed in anyone claims 1 to 3, wherein the purity of the compound N- (2,3- Dihydrobenzo[1,4]dioxin-2-carbonyl) piperazine obtained is in the range of 99.3-99.9%.

## Patentansprüche

1. Verfahren zur Herstellung von N-(2,3-Dihydrobenzo[1,4]-dioxin-2-carbonyl)piperazin, das das Erhitzen eines Reaktionsgemischs von 2,3-Dihydrobenzo[1,4]dioxin-2-carboxylat und Piperazin mit einem Molverhältnis von Carboxylat zu Piperazin im Bereich von 1:2 bis 2:1 unter Stickstoffatmosphäre bei einer Temperatur im Bereich von 70 - 90 °C während eines Zeitraums von 3 - 15 h, das Abkühlen des obigen Reaktionsgemischs auf eine Temperatur von 25 - 30 °C und das Lösen desselben in Chloroform und anschließend das Waschen mit jeweils gesättigter Natriumbicarbonatlösung und Wasser, das Ansäuern der organischen Schicht mit etwa 10 %iger HCl auf einen pH-Wert im Bereich zwischen 1 und 4, das Abtrennen der organischen Schicht und das Waschen der wässrigen Schicht mit Chloroform, das Basischmachen der wässrigen Schicht mit Natriumbicarbonat auf einen pH-Wert im Bereich zwischen 7 und 9 und anschließend das Extrahieren mit Chloroform und Abdampfen des Chloroforms, wobei das gewünschte Endprodukt in amorpher fester Form erhalten wird, umfasst.

2. Verfahren gemäß Anspruch 1, wobei das Reaktionsgemisch während eines Zeitraums von vorzugsweise im Bereich von 5 - 12 h erhitzt wird.

3. Verfahren gemäß einem der Ansprüche 1 und 2, wobei die verwendete Reaktionstemperatur vorzugsweise im Bereich von 75 - 80 °C liegt.

4. Verfahren gemäß einem der Ansprüche 1 bis 3, wobei die Reinheit der erhaltenen Verbindung N-(2,3-Dihydrobenzo-[1,4]dioxin-2-carbonyl)piperazin im Bereich von 99,3 - 99,9 % liegt.

## Revendications

1. Procédé pour la préparation de la N-(2,3-dihydrobenzo[1,4]dioxin-2-carbonyl)pipérazine, qui comprend les étapes consistant à chauffer un mélange réactionnel de 2,3-dihydrobenzo[1,4]dioxin-2-carboxylate et de pipérazine avec un rapport molaire du carboxylate à la pipérazine dans la gamme de 1:2 à 2:1, sous une atmosphère d'azote, à une température dans la gamme de 70 à 90°C pendant une durée de 3 à 15 heures, à refroidir ledit mélange réactionnel ci-dessus jusqu'à une température de 25 à 30°C et à le dissoudre dans le chloroforme puis le laver avec une solution de bicarbonate de sodium saturée et de l'eau, respectivement, à acidifier la couche organique par HCl à 10 % environ jusqu'à un pH compris entre 1 et 4, à séparer la couche organique et laver la couche aqueuse avec du chloroforme, à rendre basique la couche aqueuse avec du bicarbonate de sodium jusqu'à un pH compris entre 7 et 9 puis à l'extraire avec du chloroforme et à évaporer le chloroforme pour obtenir le produit final souhaité sous forme solide amorphe.

2. Procédé selon la revendication 1, dans lequel le mélange réactionnel est chauffé pendant une durée de préférence dans la gamme de 5 à 12 heures.

3. Procédé selon l'une quelconque des revendications 1 et 2, dans lequel la température de réaction utilisée est de préférence dans la gamme de 75 à 80°C.

4. Procédé selon l'une quelconque des revendications 1 à 3, dans lequel la pureté du composé de N-(2,3-dihydrobenzo[1,4]dioxin-2-carbonyl)pipérazine obtenu est dans la gamme de 99,3 à 99,9 %.
